(19) Europäisches Patentamt
European Patent Office
Office européen des brevets

(11) **EP 4 644 370 A1**

(12) **EUROPEAN PATENT APPLICATION**
published in accordance with Art. 153(4) EPC

(43) Date of publication:
**05.11.2025 Bulletin 2025/45**

(21) Application number: **23912461.3**

(22) Date of filing: **20.09.2023**

(51) International Patent Classification (IPC):
***C07D 209/30*** (2006.01) ***C07D 209/34*** (2006.01)
***C07D 405/12*** (2006.01) ***C07D 401/12*** (2006.01)
***C07D 409/12*** (2006.01) ***C09J 11/06*** (2006.01)
***C09J 7/30*** (2018.01) ***C08K 5/3417*** (2006.01)
***C08K 5/45*** (2006.01) ***C08K 5/372*** (2006.01)

(52) Cooperative Patent Classification (CPC):
**C07D 209/30; C07D 209/34; C07D 401/12; C07D 405/12; C07D 409/12; C08F 2/50; C08F 220/06; C08F 220/32; C08F 265/06; C08K 5/3417; C08K 5/372; C08K 5/378; C08K 5/45; C08L 33/06; C09J 7/30;** (Cont.)

(86) International application number:
**PCT/KR2023/014221**

(87) International publication number:
**WO 2024/143778 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **26.12.2022 KR 20220184448**

(71) Applicant: **LG CHEM, LTD.**
**Seoul 07336 (KR)**

(72) Inventors:
- **SON, Seonkyoung**
**Daejeon 34122 (KR)**
- **LE, Duy Hieu**
**Daejeon 34122 (KR)**
- **LEE, Hoyong**
**Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **COMPOUND, ADHESIVE COMPOSITION COMPRISING SAME, ADHESIVE FILM, AND ELECTRONIC DEVICE**

(57) The present invention relates to a compound, an adhesive composition including the same, and an adhesive film and an electronic device including the same. The compound according to one embodiment of the present invention has the structure of Chemical Formula 1, and provides an advantage of improving light resistance characteristics due to the structure.

[Figure 1]

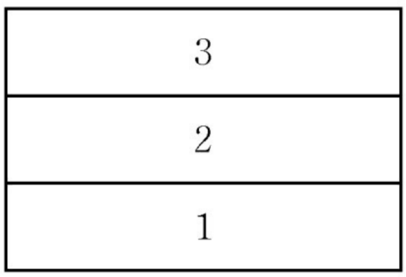


EP 4 644 370 A1

(52) Cooperative Patent Classification (CPC): (Cont.)
**C09J 11/06; G03F 7/004; G03F 7/033**

## Description

[Technical Field]

The present invention relates to a compound, and an adhesive composition, an adhesive film and an electronic device including the same.

<Cross-Reference to Related Applications>

This application claims priority to and the benefit of Korean Patent Application No. 10-2022-0184448 filed in the Korean Intellectual Property Office on December 26, 2022, the entire contents of which are incorporated herein by reference.

[Background Art]

A display device included in an electronic device typically implements an image with a combination of lights having a specific wavelength of R (Red), G (Green), and B (Blue). In this case, in the case of indoors, the visibility of the display device is good because there is no external light having high energy. However, in the case of outdoors, strong light reflection occurs on the surface of the device, electrodes in the device, and the like due to external light having high energy such as sunlight, and accordingly, the amount of light emitted from the display is reduced, so that there is a problem in that the contrast ratio and visibility of the display are significantly reduced.

In order to solve the problems of a reduction in the contrast ratio and a reduction in visibility due to the reflection of external light in the display, there has been an attempt to reduce the reflected light by designing a display so as to include a region that absorbs external light in the display. For example, in the case of an organic light emitting device (OLED), a polarizing plate is included in the device in order to reduce the reflected light due to the aforementioned external light. The polarizing plate included in the electronic device improves surface reflection, electrode reflection, and image emission brightness by adjusting the amount of external light absorbed.

However, when a polarizing plate is used for an electronic device to adjust the amount of external light, there are problems in that the hue of light emitting color cannot be flexibly adjusted, the material cost is increased, and the device structure cannot be flexibly designed.

[Citation List]

(Patent Document 1) Japanese Patent Application Laid-Open No. 2012-211305

[Detailed Description of the Invention]

[Technical Problem]

To solve the above problems, the present specification has been made in an effort to provide a compound of the following Chemical Formula 1.

[Chemical Formula 1]

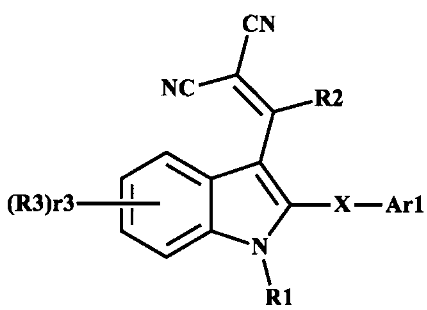

The present invention has been made in an effort to provide an adhesive composition including the compound, and an adhesive film and an electronic device including the same.

[Technical Solution]

An exemplary embodiment of the present invention provides a compound represented by the following Chemical Formula 1.

[Chemical Formula 1]

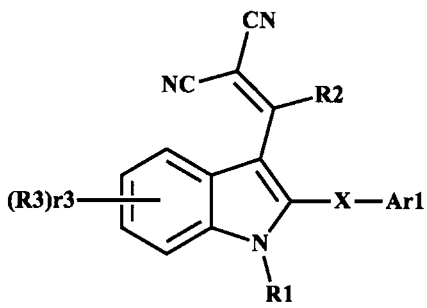

In Chemical Formula 1,

R1 to R3 are the same as or different from each other, and are each independently hydrogen; a halogen group; or a substituted or unsubstituted alkyl group,
X is O or S,
Ar1 is a substituted or unsubstituted aryl group; or a substituted or unsubstituted heteroaryl group, and
r3 is an integer from 0 to 4, and when r3 is 2 or higher, R3's are the same as or different from each other.

Another exemplary embodiment of the present invention provides an adhesive composition including the above-described compound; and an adhesive material.
Still another exemplary embodiment of the present invention provides an adhesive film including the adhesive composition.
Finally, yet another exemplary embodiment of the present invention provides an electronic device including the adhesive film.

[Advantageous Effects]

The compound according to an exemplary embodiment of the present invention and/or an adhesive composition including the same strongly absorb(s) light in a specific wavelength region to improve the light blocking efficiency.
The compound according to an exemplary embodiment of the present invention and/or an adhesive composition including the same reduce(s) reflectance to external light to improve light resistance characteristics.

[Brief Description of Drawings]

FIG. 1 illustrates an adhesive film including the adhesive composition according to an exemplary embodiment of the present invention.

[Explanation of Reference Numerals and Symbols]

1: Glass
2: Adhesive film
3: Binder resin film

[Best Mode]

Hereinafter, the present invention will be described in detail.
When one member (layer) is disposed "on" another member (layer) in the present invention, this includes not only a case where the one member (layer) is brought into contact with another member, but also a case where still another

member (layer) is present between the two members (layers).

When one part "includes" one constituent element in the present invention, unless otherwise specifically described, this does not mean that another constituent element is excluded, but means that another constituent element may be further included.

In the present invention, the "layer" has a meaning compatible with a "film" usually used in the art, and means a coating covering a target region. The size of the "layer" is not limited, and the sizes of the respective "layers" may be the same as or different from one another. According to an exemplary embodiment, the size of the "layer" may be the same as that of the entire device, may correspond to the size of a specific functional region, and may also be as small as a single sub-pixel.

Unless otherwise defined in the present invention, all technical and scientific terms used in the present invention have the same meaning as commonly understood by one with ordinary skill in the art to which the present invention pertains. Although methods and materials similar to or equivalent to those described in the present invention may be used in the practice or in the test of exemplary embodiments of the present invention, suitable methods and materials will be described below. All publications, patent applications, patents, and other references mentioned in the present invention are hereby incorporated by reference in their entireties, and in the case of conflict, the present invention, including definitions, will control unless a particular passage is mentioned. In addition, the materials, methods, and examples are illustrative only and are not intended to be limiting.

In the present invention, the term "combination thereof" included in the Markush type expression means a mixture or combination of one or more selected from the group consisting of constituent elements described in the Markush type expression, and means including one or more selected from the group consisting of the above-described constituent elements.

Examples of the substituents in the present invention will be described below, but are not limited thereto.

In the present invention, "

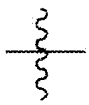

" means a moiety to be each linked.

In the present invention, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present invention, the term "substituted or unsubstituted" means being substituted with one or more substituents selected from the group consisting of deuterium; a halogen group; a nitro group; a nitrile group; $-SO_3^-$; a sulfonyl group; $-CO_2^-$; an ether group; an ester group; an alkyl group; a cycloalkyl group; an amine group; an aryl group; and a heteroaryl group, being substituted with a substituent to which two or more substituents among the exemplified substituents are linked, or having no substituent.

In the present invention, the fact that two or more substituents are linked indicates that hydrogen of any one substituent is linked to another substituent. For example, an isopropyl group and a phenyl group may be linked to each other to become a substituent of

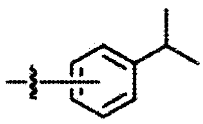

or

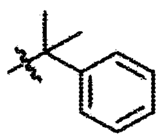

.

In the present invention, the fact that three substituents are linked to one another may include not only a case where (Substituent 1)-(Substituent 2)-(Substituent 3) are consecutively linked to one another, but also a case where (Substituent 2) and (Substituent 3) are linked to (Substituent 1). For example, two phenyl groups and an isopropyl group may be linked to each other to become a substituent of

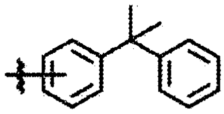

or

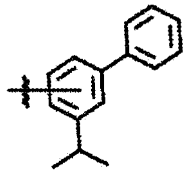

.

The same also applies to the case where four or more substituents are linked to one another.

In the present invention, hydrogen is light hydrogen ($^1H$) or may be its isotope deuterium ($^2H$) or tritium ($^3H$).

In the present invention, a halogen group is a fluoro group (-F), a chloro group (-Cl), a bromo group (-Br), or an iodo group (-I).

In the present invention, a nitro group is $-NO_2$.

In the present invention, a nitrile group is -CN, coordinates to a metal, is easily converted between a coordinated state and a free non-coordinated state, and is a non-ionic functional group.

In the present invention, a sulfonyl group may be-$SO_2R_{sulfonyl}$, and $R_{sulfonyl}$ may be selected from the group consisting of hydrogen; deuterium; a hydroxyl group; a halogen group; a nitrile group; a substituted or unsubstituted monocyclic or polycyclic cycloalkyl group having 3 to 30 carbon atoms; a substituted or unsubstituted straight or branched alkyl group having 1 to 30 carbon atoms; a substituted or unsubstituted monocyclic or polycyclic aryl group having 6 to 30 carbon atoms; and a substituted or unsubstituted monocyclic or polycyclic heteroaryl group having 2 to 30 carbon atoms. Further, the sulfonyl group may include a sulfonic acid group. For example, when $R_{sulfonyl}$ is a hydroxyl group, the sulfonyl group becomes $-SO_3H$ meaning a sulfonic acid group, and accordingly, the sulfonyl group includes a sulfonic acid group.

In the present invention, an ether group may be-$OR_{ETER}$, and $R_{ETER}$ may be selected from the group consisting of hydrogen; deuterium; a hydroxyl group; a halogen group; a nitrile group; a substituted or unsubstituted monocyclic or polycyclic cycloalkyl group having 3 to 30 carbon atoms; a substituted or unsubstituted straight or branched alkyl group having 1 to 30 carbon atoms; a substituted or unsubstituted monocyclic or polycyclic aryl group having 6 to 30 carbon atoms; and a substituted or unsubstituted monocyclic or polycyclic heteroaryl group having 2 to 30 carbon atoms.

In the present invention, an ester group may be-$COOR_{ESTER}$, and $R_{ESTER}$ may be selected from the group consisting of hydrogen; deuterium; a hydroxyl group; a halogen group; a nitrile group; a substituted or unsubstituted monocyclic or polycyclic cycloalkyl group having 3 to 30 carbon atoms; a substituted or unsubstituted straight or branched alkyl group having 1 to 30 carbon atoms; a substituted or unsubstituted monocyclic or polycyclic aryl group having 6 to 30 carbon atoms; and a substituted or unsubstituted monocyclic or polycyclic heteroaryl group having 2 to 30 carbon atoms.

In the present invention, an alkyl group may be straight-chained or branched, and the number of carbon atoms thereof is not particularly limited, but is preferably 1 to 30; 1 to 20; 1 to 10; or 1 to 5. Specific examples thereof include methyl, ethyl, propyl, n-propyl, isopropyl, butyl, n-butyl, isobutyl, t-butyl, sec-butyl, 1-methylbutyl, 1-ethylbutyl, pentyl, n-pentyl, isopentyl, neopentyl, t-pentyl, hexyl, n-hexyl, 1-methylpentyl, 2-methylpentyl, 3,3-dimethylbutyl, 2-ethylbutyl, heptyl, n-heptyl, 1-methylhexyl, cyclopentylmethyl, cyclohexylmethyl, octyl, n-octyl, t-octyl, 1-methylheptyl, 2-ethylhexyl, 2-propylpentyl, n-nonyl, 2,2-dimethylheptyl, 1-ethylpropyl, 1,1-dimethylpropyl, isohexyl, 4-methylhexyl, 5-methylhexyl, and the like, but are not limited thereto.

The alkyl group may include an alkyl group substituted with a halogen group. Specific examples thereof include a methyl group substituted with three fluoro groups, that is, a trifluoromethyl group, and the like, but are not limited thereto.

In the present invention, a cycloalkyl group is not particularly limited, but the number of carbon atoms thereof is not particularly limited, but is preferably 3 to 60; 3 to 30; 3 to 20; or 3 to 10. Specific examples of the cycloalkyl group include a cyclopropyl group, a cyclobutyl group, a cyclopentyl group, a cyclohexyl group, a cycloheptyl group, a cyclooctyl group, and the like, but are not limited thereto.

In the present invention, an aryl group means a monovalent aromatic hydrocarbon or a monovalent group of an aromatic hydrocarbon derivative. In the present invention, an aromatic hydrocarbon means a compound in which pi electrons are completely conjugated and which contains a planar ring, and a group derived from an aromatic hydrocarbon means a structure in which an aromatic hydrocarbon or a cyclic aliphatic hydrocarbon is fused with an aromatic hydrocarbon. Further, in the present invention, an aryl group intends to include a monovalent group in which two or more aromatic hydrocarbons or derivatives of an aromatic hydrocarbon are linked to each other. The aryl group is not particularly limited, but preferably has 6 to 60 carbon atoms; 6 to 50 carbon atoms; 6 to 30 carbon atoms; 6 to 25 carbon atoms; 6 to 20 carbon atoms; 6 to 18 carbon atoms; 6 to 15 carbon atoms; 6 to 13 carbon atoms; or 6 to 12 carbon atoms, and may be a monocyclic aryl group or a polycyclic aryl group.

The monocyclic aryl group is not particularly limited, but preferably has 6 to 60 carbon atoms; 6 to 54 carbon atoms; 6 to 48 carbon atoms; 6 to 42 carbon atoms; 6 to 36 carbon atoms; 6 to 30 carbon atoms; 6 to 24 carbon atoms; 6 to 18 carbon atoms; or 6 to 12 carbon atoms, and may be specifically a phenyl group, a biphenyl group, a terphenyl group, and the like, but is not limited thereto.

The polycyclic aryl group is not particularly limited, but preferably has 6 to 60 carbon atoms; 6 to 45 carbon atoms; 6 to 30 carbon atoms; 6 to 22 carbon atoms; ; 6 to 20 carbon atoms; 6 to 18 carbon atoms; 6 to 16 carbon atoms; 6 to 15 carbon atoms; 6 to 14 carbon atoms; 6 to 13 carbon atoms; 6 to 12 carbon atoms; or 6 to 10 carbon atoms, and may be a naphthyl group, an anthracenyl group, a phenanthrenyl group, a pyrenyl group, a perylenyl group, a triphenylenyl group, a chrysenyl group, a fluorenyl group, and the like, but is not limited thereto.

In the present invention, a heteroaryl group means a monovalent aromatic hetero ring. Here, the aromatic hetero ring is a monovalent group of an aromatic ring or a derivative of the aromatic ring, and means a group including one or more selected from the group consisting of N, O, P, S, Si and Se as a heteroatom. The derivative of the aromatic ring includes all structures in which an aromatic ring or an aliphatic ring is fused with an aromatic ring. Further, in the present specification, the heteroaryl group intends to include a monovalent group in which two or more aromatic rings including a heteroatom or two or more derivatives of an aromatic ring including a heteroatom are linked to each other. The number of carbon atoms of the heteroaryl group is preferably 2 to 60; 2 to 50; 2 to 30; 2 to 20; 2 to 18; or 2 to 13. Examples of the heteroaryl group include a thiophene group, a furanyl group, a pyrrole group, an imidazole group, a thiazole group, an oxazole group, a pyridine group, a pyrimidine group, a triazine group, a triazole group, an acridine group, a pyridazine group, a pyrazine group, a quinoline group, a quinazoline group, a quinoxaline group, an isoquinoline group, an indole group, a carbazole group, a benzoxazole group, a benzimidazole group, a benzothiazole group, a benzocarbazole group, a benzothiophene group, a dibenzothiophene group, a benzofuran group, a phenanthrolinyl group, a dibenzofuran group, and the like, but are not limited thereto.

In the present invention, the heteroaryl group may be monocyclic or polycyclic, and may be an aromatic ring, an aliphatic ring, or a fused ring of the aromatic ring and the aliphatic ring.

In the present invention, the heterocyclic group is a monovalent group of an aliphatic ring, a derivative of the aliphatic ring, an aromatic ring or a derivative of the aromatic ring, and means a group including one or more selected from the group consisting of N, O, P, S, Si and Se as a heteroatom.

In the present invention, the aliphatic ring is not an aromatic but a hydrocarbon ring, and examples thereof include examples of the above-described cycloalkyl group, an adamantyl group, and the like.

In the present invention, the above-described content on the aryl group may be applied to an aromatic ring.

In the present invention, the "adjacent" group may mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring may be interpreted as groups "which are adjacent" to each other.

In the present invention, in a substituted or unsubstituted ring formed by bonding groups, the "ring" means a hydrocarbon ring; or a hetero ring.

In the present invention, a hydrocarbon ring may be an aromatic ring, an aliphatic ring, or a fused ring of the aromatic ring and the aliphatic ring. Examples of the fused ring of the aromatic ring and the aliphatic ring include a 1,2,3,4-tetrahydronaphthalene group, a 2,3-dihydro-1H-indene group, and the like, but are not limited thereto.

In the present invention, the above-described description on the heterocyclic group may be applied to a hetero ring except that the hetero ring is a divalent group.

In the present invention, the above-described description on the aryl group may be applied to an aromatic hydrocarbon ring except that the aromatic hydrocarbon ring is a divalent group.

In the present invention, the above-described description on the cycloalkyl group may be applied to an aliphatic hydrocarbon ring except that the aliphatic hydrocarbon ring is a divalent group.

In the present invention, the group in the parenthesis means a substituted group. For example, -N(Rk) means -N substituted with Rk. As another example, -C(= O)Rk means that -C and O are linked by a double bond and -C is linked to Rk. As still another example, -C(=O)ORk means that -C and any one O are linked by a double bond, -C is linked to the other O, and the other O is linked to Rk.

In the present invention, Me means a methyl group ($-CH_3$), Et means an ethyl group ($-C_2H_5$), tBu means a tert-butyl group, Ph means a phenyl group, and Cy means a cyclohexyl group.

## <Compound>

An exemplary embodiment of the present invention provides a compound represented by the following Chemical Formula 1.

[Chemical Formula 1]

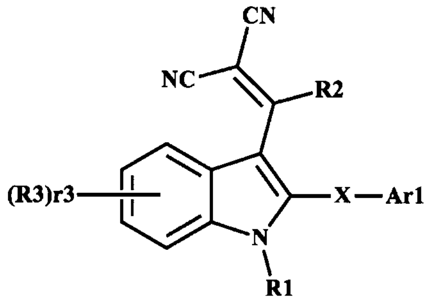

In Chemical Formula 1,

R1 to R3 are the same as or different from each other, and are each independently hydrogen; a halogen group; or a substituted or unsubstituted alkyl group,
X is O or S,
Ar1 is a substituted or unsubstituted aryl group; or a substituted or unsubstituted heteroaryl group, and
r3 is an integer from 0 to 4, and when r3 is 2 or higher, R3's are the same as or different from each other.

According to an exemplary embodiment of the present invention, the compound may be used in an electronic device or a film included in an electronic device instead of a polarizing plate. Accordingly, the material cost may be reduced or the flexibility of the device structure design may be increased compared to the case where the polarizing plate is used. In addition, when the compound is used in an optical film and/or an electronic device including the optical film instead of a polarizing plate, a high power for reinforcing low brightness is not required because it is possible to exclude a factor in which brightness is reduced by a circular polarizing plate when a polarizing plate is used.
Furthermore, in order to adjust the amount of light exposed to an electronic device, the compound according to an exemplary embodiment of the present invention used instead of a polarizing plate may adjust not only electrode reflection occurring on the electrode part in the device, but also surface reflection occurring on the surface of the device, so that the hue of light emitting color may be flexibly adjusted compared to the polarizing plate.
Further, the compound is represented by Chemical Formula 1, and specifically, the compound has a structure in which in an aromatic group (aryl group or heteroaryl group) is linked to carbon 2 via an O or S linker, and a geminal-substituted vinyl group (that is, malononitrile) is linked to carbon 3 in an indole structure, and has excellent light resistance, heat resistance, and moisture resistance due to the structure, and may strongly absorb light in a specific wavelength region. Specifically, the compound of Chemical Formula 1 includes malonitrile, and may strongly absorb light in a wavelength range of 380 nm to 420 nm due to the inclusion of malonitrile.
According to an exemplary embodiment of the present specification, R1 to R3 are the same as or different from each other, and may be each independently hydrogen; a halogen group; or a substituted or unsubstituted alkyl group having 1 to 30 carbon atoms.
According to an exemplary embodiment of the present specification, R1 to R3 are the same as or different from each other, and may be each independently hydrogen; a halogen group; or a substituted or unsubstituted alkyl group having 1 to 20 carbon atoms.
According to an exemplary embodiment of the present specification, R1 to R3 are the same as or different from each other, and may be each independently hydrogen; a halogen group; or a substituted or unsubstituted alkyl group having 1 to 10 carbon atoms.
According to an exemplary embodiment of the present invention, examples of R1 to R3 include hydrogen, methyl, ethyl, propyl, n-propyl, isopropyl, butyl, n-butyl, isobutyl, t-butyl, sec-butyl, 1-methylbutyl, 1-ethylbutyl, pentyl, n-pentyl, isopentyl, neopentyl, t-pentyl, hexyl, n-hexyl, 1-methylpentyl, 2-methylpentyl, 3,3-dimethylbutyl, 2-ethylbutyl, heptyl, n-heptyl, 1-methylhexyl, cyclopentylmethyl, cyclohexylmethyl, octyl, n-octyl, t-octyl, 1-methylheptyl, 2-ethylhexyl, 2-propylpentyl, n-nonyl, 2,2-dimethylheptyl, 1-ethylpropyl, 1,1-dimethylpropyl, isohexyl, 4-methylhexyl, 5-methylhexyl, and the like, but are not limited thereto.
According to an exemplary embodiment of the present invention, R1 may be hydrogen; or a substituted or unsubstituted alkyl group having 1 to 30 carbon atoms.
According to an exemplary embodiment of the present invention, R1 may be hydrogen; or a substituted or unsubstituted alkyl group having 1 to 20 carbon atoms.
According to an exemplary embodiment of the present invention, R1 may be hydrogen; or a substituted or unsubstituted alkyl group having 1 to 10 carbon atoms.

According to an exemplary embodiment of the present invention, R1 may be hydrogen, methyl, ethyl, or isobutyl.

According to an exemplary embodiment of the present invention, R2 may be hydrogen.

According to an exemplary embodiment of the present invention, R3 may be hydrogen; a halogen group; or a substituted or unsubstituted alkyl group having 1 to 30 carbon atoms.

According to an exemplary embodiment of the present invention, R3 may be hydrogen; a halogen group; or a substituted or unsubstituted alkyl group having 1 to 20 carbon atoms.

According to an exemplary embodiment of the present invention, R3 may be hydrogen; a halogen group; or a substituted or unsubstituted alkyl group having 1 to 10 carbon atoms.

According to an exemplary embodiment of the present invention, R3 may be hydrogen, a halogen group, or a methyl group.

According to an exemplary embodiment of the present invention, r3 may be 0.

According to an exemplary embodiment of the present invention, r3 may be 1.

According to an exemplary embodiment of the present invention, r3 may be 2.

According to an exemplary embodiment of the present invention, r3 may be 3.

According to an exemplary embodiment of the present invention, r3 may be 4.

According to an exemplary embodiment of the present invention, Ar1 may be a substituted or unsubstituted aryl group having 6 to 60 carbon atoms; or a substituted or unsubstituted heteroaryl group having 2 to 60 carbon atoms.

According to an exemplary embodiment of the present invention, Ar1 may be a substituted or unsubstituted aryl group having 6 to 30 carbon atoms; or a substituted or unsubstituted heteroaryl group having 2 to 30 carbon atoms.

According to an exemplary embodiment of the present invention, Ar1 may be a substituted or unsubstituted aryl group having 6 to 20 carbon atoms; or a substituted or unsubstituted heteroaryl group having 2 to 20 carbon atoms.

According to an exemplary embodiment of the present invention, Ar1 may be a substituted or unsubstituted phenyl group, an unsubstituted pyridine group, a substituted or unsubstituted furan group, or a substituted or unsubstituted thiophene group.

According to an exemplary embodiment of the present invention, when Ar1 is a substituted phenyl group, a substituent thereof is selected from an alkyl group, a tert-butyl group, a methoxy group and a nitro group, or two or more adjacent substituents may be linked to each other to form an α, β-unsaturated lactone ring.

According to an exemplary embodiment of the present invention, when Ar1 is a substituted furan group, two or more adjacent substituents may be linked to each other to form a phenyl ring, thereby forming a benzofuran group.

According to an exemplary embodiment of the present invention, when Ar1 is a substituted thiophene group, a substituent thereof may be an alkyl group.

According to an exemplary embodiment of the present invention, Chemical Formula 1 may be represented by any one of the following compounds.

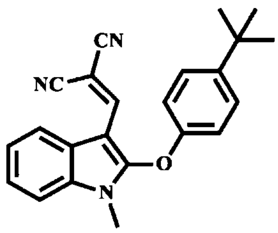
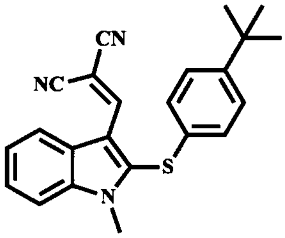
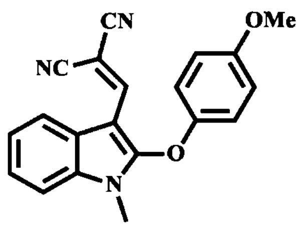

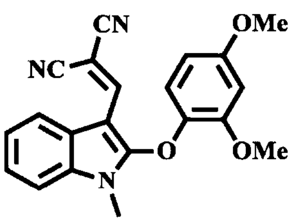
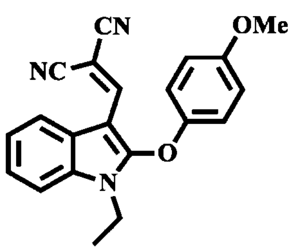
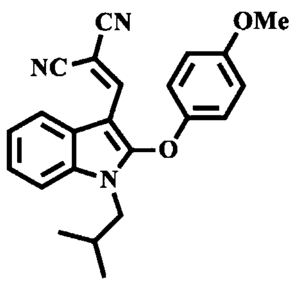

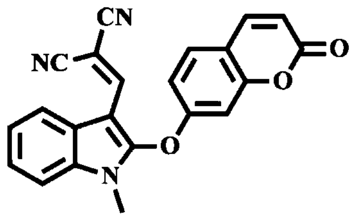
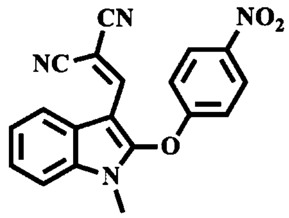

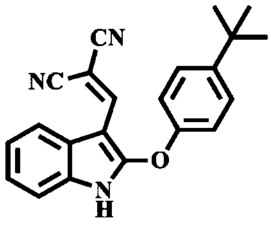
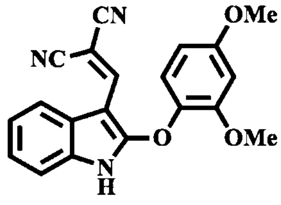
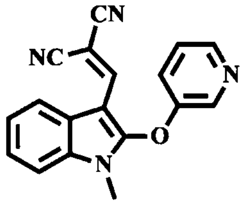

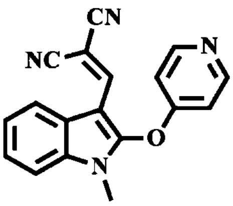
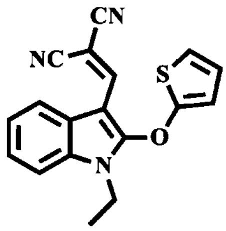
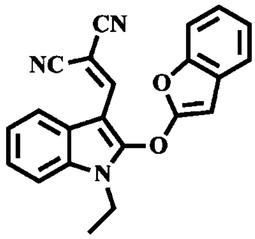

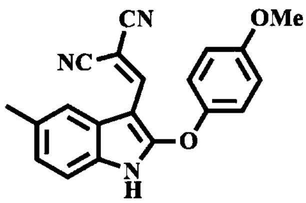
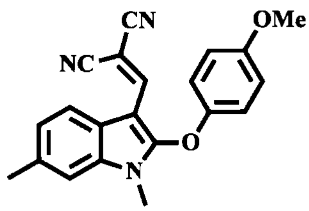
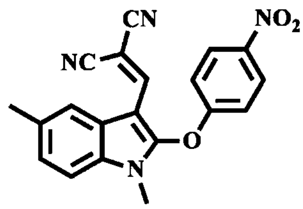

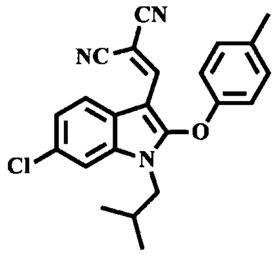
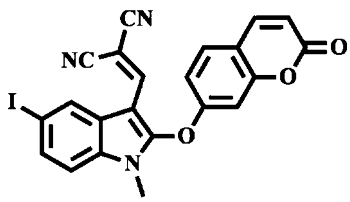
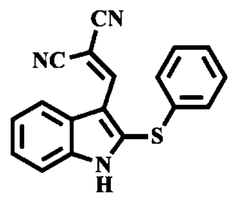

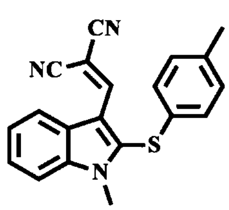
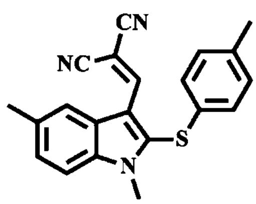
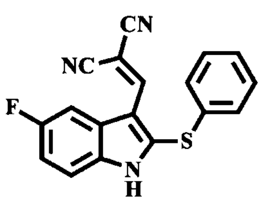

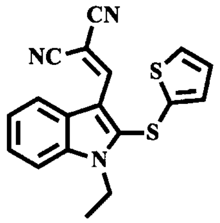

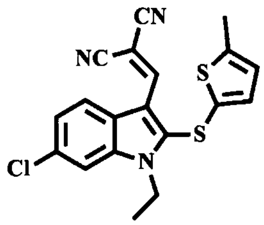

However, the compound is the exemplary compound of the present invention, and is not limited thereto.

The compound of Chemical Formula 1 of the present specification may be prepared by the following Reaction Scheme. The substituent may be bonded by a method known in the art, and the type and position of the substituent and the number of substituents may be changed according to the technology known in the art.

[Reaction Scheme]

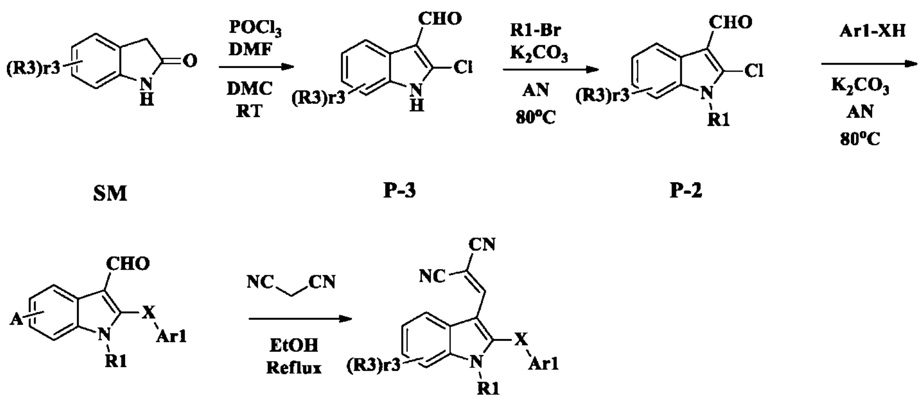


SM P-3 P-2

P-1 P

In the Reaction Scheme, the definition of each substituent is the same as that described above.

In the present invention, the above-described compound is easily synthesized by a known method, or a commercially available material or a derivative thereof may be used. The known method is not particularly limited as long as a desired compound can be obtained.

**<Adhesive composition>**

An exemplary embodiment of the present invention provides an adhesive composition (also referred to as PSA (pressure-sensitive adhesive)) including the above-described compound.

An exemplary embodiment of the present invention provides an adhesive composition including the above-described compound; and an adhesive material.

According to an exemplary embodiment of the present invention, a publicly-known adhesive material typically used in the art can be used as long as the adhesive material is a material that can be used for a film for a display while imparting adhesiveness. Examples of the adhesive material include an acrylic polymer, an acrylate-based copolymer, a silicone-based polymer, polyester, polyurethane, polyether, an epoxy resin, and the like, but are not limited thereto.

According to an exemplary embodiment of the present invention, the adhesive material may be an acrylate-based resin. The acrylate-based resin may be an acrylate-based polymer or an acrylate-based copolymer. Examples of the acrylate-based copolymer may include butyl acrylate/hydroxyethyl acrylate, but are not limited to the examples.

According to preferred exemplary embodiments of the present invention, as the adhesive material, AD-701 manufactured by LG Chem. may be used, but the adhesive material is not limited thereto.

According to an exemplary embodiment of the present invention, the adhesive material may be included in an amount of 93 to 97 parts by weight; or 94 to 96 parts by weight based on 100 parts by weight of a solid content except for a solvent in the adhesive composition. According to a preferred exemplary embodiment of the present invention, the adhesive material may be included in an amount of 95 to 96 parts by weight based on 100 parts by weight of a solid content except for a solvent in the adhesive composition.

According to an exemplary embodiment of the present invention, the compound may be included in an amount of

0.10 to 0.50 parts by weight; or 0.20 to 0.30 parts by weight based on 100 parts by weight of a solid content except for a solvent in the adhesive composition. According to a preferred exemplary embodiment of the present invention, the compound may be included in an amount of 0.25 to 0.30 parts by weight based on 100 parts by weight of a solid content except for a solvent in the adhesive composition.

According to another exemplary embodiment of the present invention, the compound may be used with other types of dyes. A dye which may be used with the compound can be used as long as the dye can be typically used in the art. As a specific example of a dye which may be used with the compound, it is possible to select one or more from the group consisting of a metal-complex-based compound; an azo-based compound; a metal azo-based compound; a quinophthalone-based compound; an isoindoline-based compound; a methine-based compound; a phthalocyanine-based compound; a metal phthalocyanine-based compound; a porphyrin-based compound; a metal porphyrin-based compound; a tetra azaporphyrin-based compound; a metal tetra aza porphyrin-based compound; a cyanine-based compound; a xanthene-based compound; a metal dipyrromethane-based compound; a boron dipyrromethane-based compound; an anthraquinone-based compound; a diketopyrrolopyrrole-based compound; a triarylmethane-based compound; and a perylene-based compound. However, the example is not limited thereto.

According to an exemplary embodiment of the present invention, the adhesive composition may further include a solvent. As the solvent, it is possible to apply a compound known to enable the formation of an adhesive composition in the art to which the present invention pertains without particular limitation. For example, the solvent may be one or more compounds selected from the group consisting of esters, ethers, ketones, aromatic hydrocarbons, and sulfoxides.

The ester solvent may be, for example, ethyl acetate, n-butyl acetate, isobutyl acetate, amyl formate, isoamyl acetate, isobutyl acetate, butyl propionate, isopropyl butyrate, ethyl butyrate, butyl butyrate, methyl lactate, ethyl lactate, gamma-butyrolactone, epsilon-caprolactone, delta-valerolactone, alkyl oxyacetate (for example: methyl oxyacetate, ethyl oxyacetate, butyl oxyacetate (for example, methyl methoxyacetate, ethyl methoxyacetate, butyl methoxyacetate, methyl ethoxyacetate, ethyl ethoxyacetate, and the like)), 3-oxypropionic acid alkyl esters (for example: methyl 3-oxypropionate, ethyl 3-oxypropionate, and the like (for example, methyl 3-methoxypropionate, ethyl 3-methoxypropionate, methyl 3-ethoxypropionate, ethyl 3-ethoxypropionate, and the like)), 2-oxypropionic acid alkyl esters (for example: methyl 2-oxypropionate, ethyl 2-oxypropionate, propyl 2-oxypropionate, and the like (for example, methyl 2-methoxypropionate, ethyl 2-methoxypropionate, propyl 2-methoxypropionate, methyl 2-ethoxypropionate, ethyl 2-ethoxypropionate)), methyl 2-oxy-2-methylpropionate and ethyl 2-oxy-2-methylpropionate (for example, methyl 2-methoxy-2-methylpropionate, ethyl 2-ethoxy-2-methylpropionate, and the like), methyl pyruvate, ethyl pyruvate, propyl pyruvate, methyl acetoacetate, ethyl acetoacetate, methyl 2-oxobutyrate, ethyl 2-oxobutyrate, or the like.

The ether solvent may be, for example, diethylene glycol dimethyl ether, tetrahydrofuran, ethylene glycol monomethyl ether, ethylene glycol monoethyl ether, methyl cellosolve acetate, ethyl cellosolve acetate, diethylene glycol monomethyl ether, diethylene glycol monoethyl ether, diethylene glycol monobutyl ether, propylene glycol monomethyl ether, propylene glycol monomethyl ether acetate, propylene glycol monoethyl ether acetate, propylene glycol monopropyl ether acetate, or the like.

The ketone solvent may be, for example, methyl ethyl ketone, cyclohexanone, cyclopentanone, 2-heptanone, 3-heptanone, N-methyl-2-pyrrolidone, or the like.

The aromatic hydrocarbon solvent may be, for example, toluene, xylene, anisole, limonene, or the like.

The sulfoxide solvent may be, for example, dimethyl sulfoxide or the like.

Although the solvent is exemplified as described above, any solvent capable of dissolving or dispersing the above-described compound of the present invention is sufficient, and the solvent is not limited to the above-exemplified solvents.

According to a preferred exemplary embodiment of the present invention, the solvent included in the adhesive composition is ethyl acetate or methyl ethyl ketone.

Further, the solvents may be used either alone or in a mixture of two or more solvents. For example, according to an exemplary embodiment of the present invention, the solvent included in the adhesive composition is a mixed solution of ethyl acetate or methyl ethyl ketone. In this case, the solvent mixed solution may be used by mixing at a ratio of 1: 3 to 3: 1, but this is merely an example, and the mixing ratio is not limited to the above example.

According to a preferred exemplary embodiment of the present invention, as the solvent, methyl ethyl ketone (MEK) may be used, but the solvent is not limited thereto.

According to an exemplary embodiment of the present invention, the solvent may be included in an amount of 10 to 50 parts by weight; or 20 to 40 parts by weight based on the total parts by weight of the adhesive composition. According to a preferred exemplary embodiment of the present invention, the solvent may be included in an amount of 20 to 30 parts by weight based on the total parts by weight of the adhesive composition, but the content is not limited thereto.

According to an exemplary embodiment of the present invention, the adhesive composition may further include other additives.

Examples of the other additives include a cross-linking agent, a silane coupling agent, a catalyst, an antioxidant, an antistatic agent, a light stabilizer, and the like, but are not limited thereto.

According to an exemplary embodiment of the present invention, the adhesive composition may further include one or more selected from among a cross-linking agent, an antioxidant, an antistatic agent, a silane coupling agent, a light stabilizer, and a catalyst.

According to an exemplary embodiment of the present invention, the cross-linking agent may induce a cross-linking reaction between an alkali-soluble polyimide resin and other additive components to increase the heat resistance and chemical resistance of a produced film. In this case, as the cross-linking agent, a compound including a functional group such as an acrylic group and an isocyanate group may be used. In addition, examples of the cross-linking agent include a thermal cross-linking agent, and as the thermal cross-linking agent, a compound including a heat-reactive functional group such as a methylol group and an epoxy group may be used. As a specific example, it is possible to use DML-PC, DML-PEP, DML-OC, DML-OEP, DML-34X, DML-PTBP, DML-PCHP, DML-OCHP, DML-PFP, DML-PSBP, DML-POP, DML-MBOC, DML-MBPC,DML-MTrisPC, DML-BisOC-Z, DML-BisOCHP-Z, DML-BPC, DML-BisOC-P, DMOM-PC, DMOM-PTBP, DMOM-MBPC, TriML-P,TriML-35XL, TML-HQ, TML-BP, TML-pp-BPF, TML-BPE, TML-BPA, TML-BPAF, TML-BPAP, TMOM-BP, TMOM-BPE, TMOM-BPA, TMOM-BPAF, TMOM-BPAP, HML-TPPHBA, HML-TPHAP, HMOM-TPPHBA, HMOM-TPHAP (all are trade names, manufactured by Honshu Chemical Industry Co., Ltd.), "NIKALAC" (registered trademark) MX-290, "NIKALAC" (registered trademark) MX-280, "NIKALAC" (registered trademark) MX-270, "NIKALAC" (registered trademark) MX-279, "NIKALAC" (registered trademark) MW-100LM, "NIKALAC" (registered trademark) MX-750LM (all are trade names, manufactured by Sanwa Chemical Co., Ltd.), T39M (Soken Chemical & Engineering Co., Ltd.), and the like, which are cross-linking agents generally used in the art.

The cross-linking agent may be included in an amount of 0.05 to 0.40 parts by weight based on 100 parts by weight of a solid content except for a solvent in the adhesive composition. As a preferred example, the cross-linking agent may be included in an amount of 0.07 to 0.40 parts by weight based on 100 parts by weight of a solid content except for a solvent in the adhesive composition, but the content is not limited thereto.

The silane coupling agent may be used, for example, to improve the dispersibility of a thermally conductive filler such as alumina, and various types of silane-based coupling agents known in the art can be used without limitation as long as the silane-based coupling agent can exhibit the action as described above. The silane-based coupling agent means a compound including a hydrolyzable silyl group or silanol group. Further, the silane-based coupling agent may increase heat resistance and chemical resistance by increasing the mutual adhesion between a film produced by curing and one specific surface of the substrate. As an example of the silane-based coupling agent, it is possible to select and use one or more from octyltrimethoxysilane, dodecyltrimethoxysilane, octadecyl trimethoxysilane, and the like, but the silane-based coupling agent is not limited thereto.

According to preferred exemplary embodiments of the present invention, as the silane coupling agent, T-789J (Soken Chemical & Engineering Co., Ltd.) may be used, but the silane coupling agent is not limited thereto.

The antioxidant may serve to prevent a chain reaction in which radicals are generated during the formation of a polymer film. In this case, the antioxidant may include a phenolic antioxidant, and the like, and 2,2-thiobis(4-methyl-6-t-butylphenol), or 2,6-g,t-butylphenol, and the like, which are antioxidants generally used in the art, may be used, and as the UV absorbent, 2-(3-t-butyl-5-methyl-2-hydroxyphenyl)-5-chloro-benzotriazole, or alkoxy benzophenone, and the like may be used, but the antioxidant and the UV absorbent are not limited thereto. According to preferred exemplary embodiments of the present invention, as the antioxidant, Kinox-80 (Hannong Chemicals Inc.) may be used, but the antioxidant is not limited thereto.

The content of the antioxidant may be 0.2 to 0.8 parts by weight; or 0.3 to 0.7 parts by weight based on 100 parts by weight of a solid content except for a solvent in the adhesive composition. As a preferred example, the antioxidant may be included in an amount of 0.4 to 0.6 parts by weight based on 100 parts by weight of a solid content except for a solvent in the adhesive composition, but the content is not limited thereto.

The antistatic agent is included in the adhesive composition, and thus serves to impart an antistatic performance, and all publicly-known antistatic agents may be used. As the antistatic agent, for example, an ionic compound may be used. As the ionic compound, for example, a metal salt or an organic salt may be used. The metal salt ionic compound may include, for example, an alkali metal cation or an alkaline earth metal cation. As the cation, one or two or more of a lithium ion ($Li^+$), a sodium ion ($Na^+$), a potassium ion ($K^+$), a rubidium ion ($Rb^+$), a cesium ion ($Cs^+$), a beryllium ion ($Be^{2+}$), a magnesium ion ($Mg^{2+}$), a calcium ion ($Ca^{2+}$), a strontium ion ($Sr^{2+}$) and a barium ion ($Ba^{2+}$) may be exemplified, and for example, it is possible to use one or two or more of a lithium ion, a sodium ion, a potassium ion, a magnesium ion, a calcium ion and a barium ion, or a lithium ion in consideration of ion stability and mobility. As the anion included in the metal salt, $PF_6^-$, $AsF^-$, $NO_2^-$, fluoride ($F^-$), chloride ($Cl^-$), bromide ($Br^-$), iodide ($I^-$), perchlorate ($ClO_4^-$), hydroxide ($OH^-$), carbonate ($CO_3^{2-}$), nitrate ($NO_3^-$), trifluoromethanesulfonate ($CF_3SO_3^-$), sulfonate ($SO_4^-$), hexafluorophosphate ($PF_6^-$), methylbenzenesulfonate ($CH_3(C_6H_4)SO_3^-$), p-toluenesulfonate ($CH_3C_6H_4SO_3^-$), tetraborate ($B_4O_7^{2-}$), carboxybenzenesulfonate ($COOH(C_6H_4)SO_3^-$), trifluoromethanesulfonate ($CF_3SO_2^-$), benzoate ($C_6H_5COO^-$), acetate ($CH_3COO^-$), trifluoroacetate ($CF_3COO^-$), tetrafluoroborate ($BF_4^-$), tetrabenzylborate ($B(C_6H_5)_4^-$), trispentafluoroethyl trifluorophosphate ($P(C_2F_5)_3F_3^-$), or the like may be exemplified. According to a preferred exemplary embodiment of the present invention, as the antistatic agent, FC-4400 (3M) may be used, but the antistatic agent is not limited thereto.

The content of the antistatic agent may be 0.5 to 2.5 parts by weight; or 0.7 to 2.3 parts by weight based on 100 parts by weight of a solid content except for a solvent in the adhesive composition. As a preferred example, the antistatic agent may be included in an amount of 1 to 2 parts by weight based on 100 parts by weight of a solid content except for a solvent in the adhesive composition, but the content is not limited thereto.

As the silane coupling agent, those known in the art may be appropriately employed, and the content of the silane coupling agent may be 0.10 to 0.80 parts by weight based on 100 parts by weight of a solid content except for a solvent in the adhesive composition. As a preferred example, the silane coupling agent may be included in an amount of 0.15 to 0.50 parts by weight based on 100 parts by weight of a solid content except for a solvent in the adhesive composition, but the content is not limited thereto.

The light stabilizer is a material which is not aggregated even when an adhesive agent is left to stand under high temperature conditions, and thus does not induce a phenomenon in which the concentration of an antistatic agent to be described below in the aggregated cluster increases, and can significantly improve the storage stability of the adhesive composition by preventing a problem in that a bonding site is degraded by heat to generate radicals, and a publicly-known light stabilizer may be used. According to a preferred exemplary embodiment of the present invention, as the light stabilizer, a hindered amine compound may be used, and specifically, Tinuvin 123 (BASF SE) may be used, but the light stabilizer is not limited thereto.

The content of the light stabilizer may be 1 to 4 parts by weight; or 1 to 3 parts by weight based on 100 parts by weight of a solid content except for a solvent in the adhesive composition. As a preferred example, the light stabilizer may be included in an amount of 1.5 to 2.5 parts by weight based on 100 parts by weight of a solid content except for a solvent in the adhesive composition, but the content is not limited thereto.

As the catalyst, a material that adjusts the rate of a reaction for preparing an adhesive composition according to an exemplary embodiment of the present invention is used. According to an exemplary embodiment of the present invention, as the catalyst, dibutyltin dilaurate (Sigma-Aldrich) may be used, but the catalyst is not limited thereto.

The content of the catalyst may be 0.005 to 0.02 parts by weight; or 0.006 to 0.019 parts by weight based on 100 parts by weight of a solid content except for a solvent in the adhesive composition. As a preferred example, the catalyst may be included in an amount of 0.007 to 0.015 parts by weight based on 100 parts by weight of a solid content except for a solvent in the adhesive composition, but the content is not limited thereto.

An exemplary embodiment of the present invention provides an adhesive composition which further includes at least one of a cross-linking agent, an antioxidant, an antistatic agent, a light stabilizer and a catalyst, in which based on 100 parts by weight of a solid content of the adhesive composition, a content of the cross-linking agent is 0.05 to 0.40 parts by weight, a content of the antioxidant is 0.20 to 0.80 parts by weight, a content of the antistatic agent is 0.50 to 2.50 parts by weight, a content of the light stabilizer is 1.00 to 4.00 parts by weight, and a content of the catalyst is 0.005 to 0.02 parts by weight.

According to an exemplary embodiment of the present invention, the adhesive composition exhibits a maximum absorption wavelength in a wavelength range of 380 nm or more and 420 nm or less.

According to an exemplary embodiment of the present invention, the maximum absorption wavelength of the adhesive composition may be about 380 nm or more, about 381 nm or more, or about 382 nm or more, or about 420 nm or less, about 410 nm or less, or about 400 nm or less.

In the present specification, the maximum absorption wavelength of the adhesive composition is measured in a solution state.

In the present specification, the maximum absorption wavelength of the adhesive composition is measured using a diluted solution at a dye concentration of $10^{-5}$ M based on the toluene solvent.

Meanwhile, the wavelength range of light mainly corresponds to the blue light in visible light. In other words, the adhesive composition may absorb blue light. Specifically, the adhesive composition may absorb blue light in the wavelength range described above and may be used as a component of a blue-cut dye.

**<Adhesive film>**

An exemplary embodiment of the present invention provides an adhesive film including the above-described adhesive composition.

According to an exemplary embodiment of the present invention, the adhesive film exhibits a maximum absorption wavelength in a wavelength range of 380 nm or more and 420 nm or less.

According to an exemplary embodiment of the present invention, the maximum absorption wavelength of the adhesive film may be about 380 nm or more, about 381 nm or more, or about 382 nm or more, or about 420 nm or less, about 410 nm or less, or about 400 nm or less.

In the present specification, for the maximum absorption wavelength of the adhesive film, a thin-film adhesive film formed by applying the above-described adhesive composition onto a base film (for example: a PET film) is used.

In the present specification, the maximum absorption wavelength of the adhesive film may be measured by

setting the thickness of the adhesive film to a range of 10 μm or more and 40 μm or less, preferably 15 μm or more and 30 μm or less, and more preferably 20 μm or more and 25 μm or less.

According to an exemplary embodiment of the present invention, the adhesive film may satisfy the following Equation (1).

$$10\ \% \geq |T_0 - T_i| \quad \text{- Equation (1)}$$

In Equation (1),

$T_0$ is a transmittance value (unit: %) measured before irradiation by coating the adhesive film with a thickness of 20±3 μm, and

$T_i$ is a transmittance value (unit: %) measured after irradiating the adhesive film with a light intensity of 55,600 lx for 48 hours.

According to an exemplary embodiment of the present invention, the adhesive film has a value of about 10 or less, about 9 or less, about 8 or less, or about 7 or less in Equation (1), meaning that the adhesive film has excellent light resistance. In the present specification, an adhesive film satisfying Equation (1) has excellent light resistance, and the closer the value of Equation (1) is to 0, the better the physical properties are secured, so that the lower limit is not particularly limited.

According to an exemplary embodiment of the present invention, an adhesive film including the above-described adhesive compositions may be used for a display, an electronic device, and the like instead of a polarizing plate to more flexibly adjust the color tone of emitted light than an optical film including a polarizing plate or improve reflected color. In particular, the adhesive film including the above-described adhesive composition has an advantage of improving the luminance transmittance for blue light.

According to an exemplary embodiment of the present invention, the adhesive film including the adhesive composition may have a form in which the adhesive composition (or the composition including the same) is laminated and/or applied onto one surface of a base film.

According to an exemplary embodiment of the present invention, the adhesive film including the adhesive composition may include the adhesive composition as it is.

According to still another exemplary embodiment of the present invention, the adhesive film including the adhesive composition may include a cured product of the adhesive composition.

According to an exemplary embodiment of the present invention, the base film may be selected from the group consisting of polyethylene terephthalate (PET), polyester, polycarbonate (PC), polyimide (PI), polyethylene naphthalate (PEN), polyether ether ketone (PEEK), polyarylate (PAR), polycycloolefin (PCO), polynorbornene, polyethersulphone (PES), and a cycloolefin polymer (COP).

Further, it is preferred that the base film is transparent. The fact that the base film is transparent described here indicates that the light transmittance of visible light in a wavelength range of 400 to 700 nm is 80% or more. When the base film has the above range, the laminated adhesive film including the adhesive composition has a characteristic that the adhesive film can be thinned.

According to an exemplary embodiment of the present invention, the adhesive film including the adhesive composition may have a thickness of 10 μm or more, 15 μm or more, 20 μm or more, or 22 μm or more, or 40 μm or less, 35 μm or less, 30 μm or less, or 25 μm or less.

According to an exemplary embodiment of the present invention, the adhesive film including the adhesive composition may have a thickness of 22 μm or more and 23 μm or less.

According to an exemplary embodiment of the present invention, the adhesive film including the adhesive composition may be prepared by applying the adhesive composition onto a base film using a bar coater.

According to an exemplary embodiment of the present invention, the adhesive film including the adhesive composition may further include other films. Examples of the other films include a release film, an anti-reflection film, a binder film, and the like.

According to an exemplary embodiment of the present invention, the release film may be included on the other surface other than one surface of the base film on which the adhesive composition is laminated and/or applied.

As the release film, a hydrophobic film may be used, the release film is a layer for protecting a film in which the adhesive composition having a very small thickness is laminated and/or applied and refers to a transparent layer which is attached to one surface of a film in which the adhesive composition is laminated and/or applied, and it is possible to use a film which is excellent in mechanical strength, heat stability, moisture shielding property, isotropy, and the like. For example, it is possible to use an acetate-based resin film such as triacetyl cellulose (TAC), a polyester-based resin film, a polyether sulfone-based resin film, a polycarbonate-based resin film, a polyamide-based resin film, a polyimide-based resin film, a polyolefin-based resin film, a cycloolefin-based resin film, a polyurethane-based resin film, an acrylic resin film, and the like, but the release film can be used as long as the release film is a commercially available silicone-treated release film, and is not limited to the above example.

According to an exemplary embodiment of the present invention, the anti-reflection film serves to improve the reflection color tone by adjusting the degree of light reflection generated from an external light source.

According to an exemplary embodiment of the present invention, the anti-reflection film has a minimum reflection wavelength of 550 nm or less; or 530 nm or less. According to a preferred exemplary embodiment of the present invention, the anti-reflection film has a minimum reflection wavelength of 500 nm or less.

A material for the anti-reflection film can be appropriately selected in order to satisfy the physical properties of the minimum reflection wavelength. For example, the anti-reflection film may include a low refractive layer. For example, the minimum reflection wavelength of the anti-reflection film tends to move to a longer wavelength as the thickness of the low refractive layer is increased, and to a shorter wavelength as the thickness of the low refractive layer is decreased. For example, the minimum reflectance of the anti-reflection film tends to decrease as the refractive index of a low refractive material decreases.

The low refractive layer may include a low refractive material. In one example, the low refractive material may be low refractive inorganic particles. In another example, low refractive inorganic particles may be silica-based particles. Examples of the silica-based particles include hollow silica, mesoporous silica, and the like, but are not limited thereto. In still another example, as the low refractive inorganic particles, magnesium fluoride ($MgF_2$) may be used.

According to an exemplary embodiment of the present invention, the low refractive layer may further include a binder resin film. The low refractive inorganic particles may be present in a dispersed state in a binder resin film.

According to an exemplary embodiment of the present invention, examples of the binder resin film include a cellulose triacetate (TAC) film, and the like, but are not limited thereto, and a publicly-known binder resin film typically used in the art may be used.

**<Electronic device>**

According to an exemplary embodiment of the present invention, provided is an electronic device including the above-described compound, the above-described adhesive composition or the above-described adhesive film.

The electronic device may include all of the interlayer insulation film of a semiconductor device, a color filter, a black matrix, an overcoat, a column spacer, a passivation film, a buffer coating film, a multilayer printed board insulation film, a cover coat of a flexible copper-clad board, a solder resist film, an insulation film of an OLED, a protection film of a thin-film transistor of a liquid crystal display device, an electrode protection film and a semiconductor protection film of an organic EL device, an OLED insulation film, an LCD insulation film, a semiconductor insulation film, a display device, and the like, but is not limited thereto.

According to an exemplary embodiment of the present invention, provided is an organic light emitting display including: an adhesive film including the above-described adhesive composition; a substrate provided on one surface of the film; and an organic light emitting layer provided on a surface opposite to a surface of the substrate brought into contact with the film.

[Mode for Invention]

Hereinafter, the present specification will be described in detail with reference to Examples for specifically describing the present specification. However, the Examples according to the present specification may be modified in various forms, and it is not interpreted that the scope of the present specification is limited to the Examples described below. The Examples of the present specification are provided to more completely explain the present specification to a person with ordinary skill in the art.

**<Synthesis Examples>**

1. Method of synthesizing Compound P-1

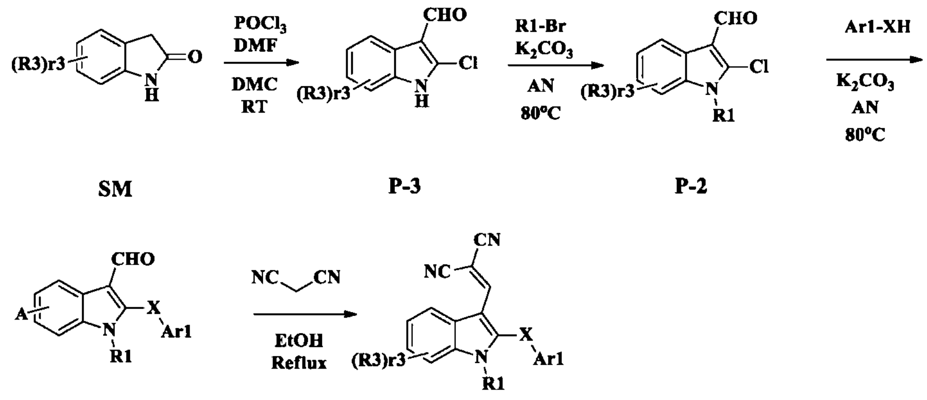


SM P-3 P-2

P-1 P

### 1) Synthesis of Compound P-3

After $POCl_3$ (30 mmol, 3 equivalents) and DMF (35 mmol, 35 mmol) were added to 20 mL of a solvent dichloromethane (DCM), 1 equivalent of oxindole (10 mmol, 1 equivalent, SM) dissolved in 20 mL of DMC was added.

After the mixed material was stirred at room temperature for 12 hours, the reaction was terminated by adding water thereto.

Then, pH was neutralized to 7 using $Na_2CO_3$, an organic layer was separated, moisture was removed using anhydrous $MgSO_4$, and then the solution was condensed.

Compound P-3 was synthesized by purifying the condensed material using a column (hexane : ethylacetate = 10 : 1).

### 2) Synthesis of Compound P-2

After Compound P-3 (10 mmol, 1 equivalent) was diluted in 150 mL of acetonitrile (AN), alkyl bromide (R1-Br, 15 mmol, 1.5 equivalents) and potassium carbonate (15 mmol, 1.5 equivalents) were added thereto, and the resulting mixture was stirred at room temperature for 10 minutes.

Then, the reaction temperature was increased to 80°C, and the reaction was allowed to proceed until Compound P-3 disappeared.

After the reaction was completed, the temperature was lowered to room temperature, water was added thereto, the resulting mixture was stirred, and then the resulting compound was synthesized by vacuum filtration.

The synthesized compound was dried in an oven and then subjected to the next reaction.

### 3) Synthesis of Compound P-1

After Compound P-2 (5 mmol, 1 equivalent) was diluted in 50 mL of acetonitrile (AN) in a reaction vessel, Ar1-XH (10 mmol, 2 equivalents) and potassium carbonate (10 mmol, 2 equivalents) were added thereto at room temperature.

The reaction temperature was increased to 80°C, the reaction was allowed to proceed until Compound P-2 disappeared, and then the temperature was lowered to room temperature, and the reaction was terminated by adding water thereto.

After the reaction mixture was extracted using ethyl acetate (EA), moisture was removed from the separated organic layer using anhydrous $MgSO_4$.

The organic layer from which moisture was removed was condensed and used for the next reaction without further purification.

## 2. Method of synthesizing Compound P

Synthesis Example 1

Compound A1 (6.5 mmol, 1 equivalent, see Table 1) was put into a reaction vessel and diluted in 50 mL of ethanol. Thereafter, malonitrile (13 mmol, 2 equivalents) was added thereto, the resulting mixture was stirred at room temperature for 10 minutes, the temperature increased to 80°C, the reaction was allowed to proceed until Compound A1 was no longer visible, and then the temperature was cooled to room temperature. The compound generated at room temperature was

collected by vacuum filtration and then dried in an oven. (1.7 g, yield 74%)

HR LC/MS/MS m/z calcd for $C_{23}H_{21}N_3O$ (M+): 355.1685; found: 355.1685

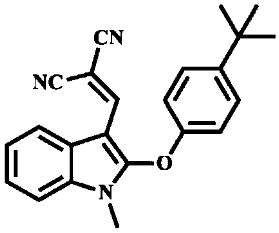

Compound 1

**Synthesis Example 2**

Compound 2 was synthesized by performing the reaction in the same manner as in Synthesis Example 1, except that Compound A2 (6.2 mmol, 1 equivalent, see Table 1) was used by replacing Compound A1. (1.86 g, yield 81%)

HR LC/MS/MS m/z calcd for $C_{23}H_{21}N_3S$ (M+): 371.1456; found: 371.1456

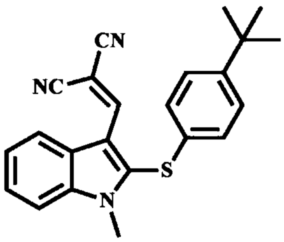

Compound 2

**Synthesis Example 3**

Compound 3 was synthesized by performing the reaction in the same manner as in Synthesis Example 1, except that Compound A3 (7.1 mmol, 1 equivalent, see Table 1) was used by replacing Compound A1. (2.06 g, yield 88%)

HR LC/MS/MS m/z calcd for $C_{20}H_{15}N_3O_2$ (M+): 329.1164; found: 329.1164

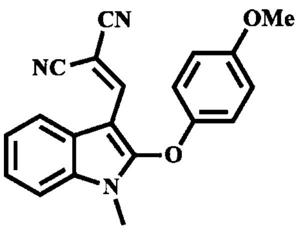

Compound 3

**Synthesis Example 4**

Compound 4 was synthesized by performing the reaction in the same manner as in Synthesis Example 1, except that Compound A4 (6.4 mmol, 1 equivalent, see Table 1) was used by replacing Compound A1. (1.89 g, yield 82%)

HR LC/MS/MS m/z calcd for $C_{21}H_{17}N_3O_3$ (M+): 359.1270; found: 359.1270

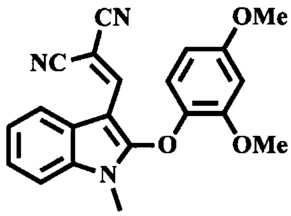

Compound 4

**Synthesis Example 5**

Compound 5 was synthesized by performing the reaction in the same manner as in Synthesis Example 1, except that Compound A5 (6.77 mmol, 1 equivalent, see Table 1) was used by replacing Compound A1. (1.99 g, yield 84%)
HR LC/MS/MS m/z calcd for $C_{21}H_{17}N_3O_2$ (M+): 343.1321; found: 343.1321

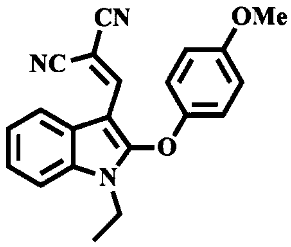

Compound 5

**Synthesis Example 6**

Compound 6 was synthesized by performing the reaction in the same manner as in Synthesis Example 1, except that Compound A6 (6.18 mmol, 1 equivalent, see Table 1) was used by replacing Compound A1. (1.72 g, yield 75%)
HR LC/MS/MS m/z calcd for $C_{23}H_{21}N_3O_2$ (M+): 371.1634; found: 371.1634

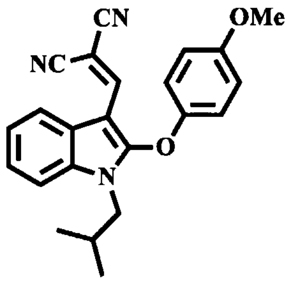

Compound 6

**Synthesis Example 7**

Compound 7 was synthesized by performing the reaction in the same manner as in Synthesis Example 1, except that Compound A7 (6.26 mmol, 1 equivalent, see Table 1) was used by replacing Compound A1. (2.0 g, yield 87%)
HR LC/MS/MS m/z calcd for $C_{22}H_{13}N_3O_3$ (M+): 367.0957; found: 367.0957

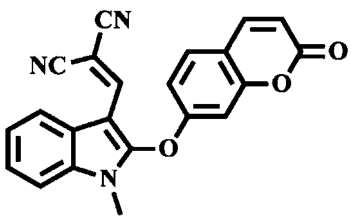

Compound 7

**Synthesis Example 8**

Compound 8 was synthesized by performing the reaction in the same manner as in Synthesis Example 1, except that Compound A8 (6.75 mmol, 1 equivalent, see Table 1) was used by replacing Compound A1. (1.58 g, yield 68%) HR LC/MS/MS m/z calcd for $C_{19}H_{12}N_4O_3$ (M+): 344.0909; found: 344.0908

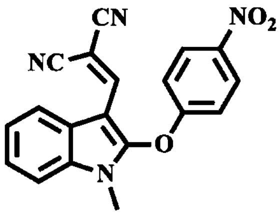

Compound 8

**Synthesis Example 9**

Compound 9 was synthesized by performing the reaction in the same manner as in Synthesis Example 1, except that Compound A9 (6.73 mmol, 1 equivalent, see Table 1) was used by replacing Compound A1. (1.44 g, yield 62%)

HR LC/MS/MS m/z calcd for $C_{20}H_{15}N_3O_3$ (M+): 345.1133; found: 345.1133

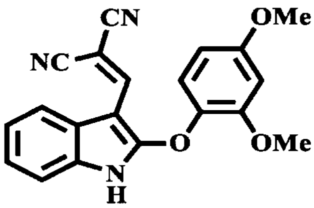

Compound 9

**Synthesis Example 10**

Compound 10 was synthesized by performing the reaction in the same manner as in Synthesis Example 1, except that Compound A10 (7.93 mmol, 1 equivalent, see Table 1) was used by replacing Compound A1. (1.29 g, yield 54%)

HR LC/MS/MS m/z calcd for $C_{18}H_{12}N_4O$ (M+): 300.1011; found: 300.1010

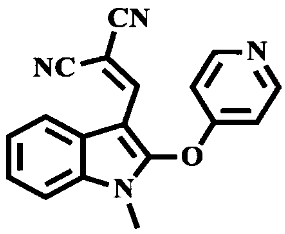

Compound 10

**Synthesis Example 11**

Compound 11 was synthesized by performing the reaction in the same manner as in Synthesis Example 1, except that Compound A11 (6.77 mmol, 1 equivalent, see Table 1) was used by replacing Compound A1. (1.74 g, yield 75%)

HR LC/MS/MS m/z calcd for $C_{21}H_{17}N_3O_2$ (M+): 343.1321; found: 343.1320

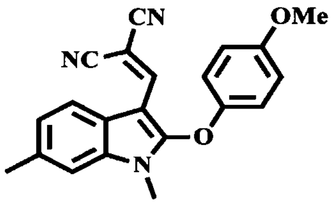

Compound 11

**Synthesis Example 12**

Compound 12 was synthesized by performing the reaction in the same manner as in Synthesis Example 1, except that Compound A12 (6.96 mmol, 1 equivalent, see Table 1) was used by replacing Compound A1. (1.49 g, yield 64%)

HR LC/MS/MS m/z calcd for $C_{18}H_{13}N_3O_2$ (M+): 335.0551; found: 335.0551

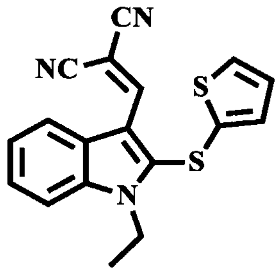

Compound 12

**Comparative Synthesis Example 1**

Comparative Compound 1 was synthesized by performing the reaction in the same manner as in Synthesis Example 1, except that Compound A13 (6.53 mmol, 1 equivalent, see Table 1) was used by replacing Compound A1. (1.85 g, yield 80%)

HR LC/MS/MS m/z calcd for $C_{23}H_{22}N_4$ (M+): 354.1844; found: 354.1844

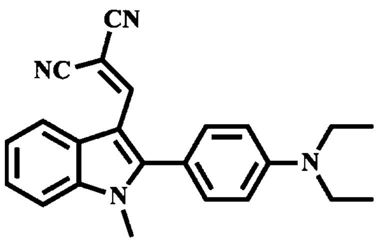

Comparative Compound 1

The starting materials used in Synthesis Examples and Comparative Synthesis Examples are shown in the following Table 1.

[Table 1]

| P-1 Compound | | P Compound | |
|---|---|---|---|
| A 1 | 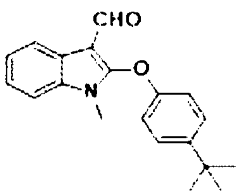 | Compound 1 | 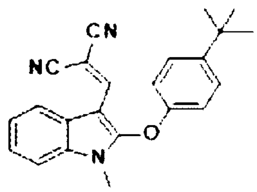 |

(continued)

| P-1 Compound | | P Compound | |
|---|---|---|---|
| A2 | 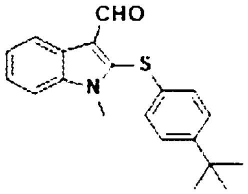 | Compound 2 | 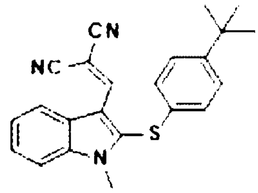 |
| A3 | 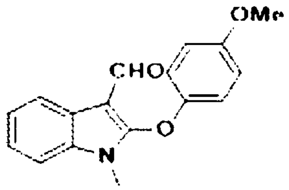 | Compound 3 | 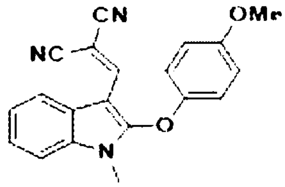 |
| A4 | 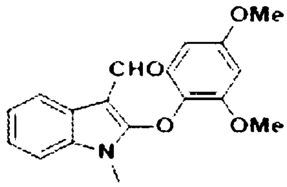 | Compound 4 | 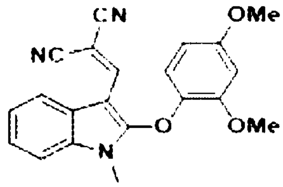 |
| A5 | 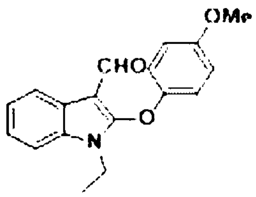 | Compound 5 | 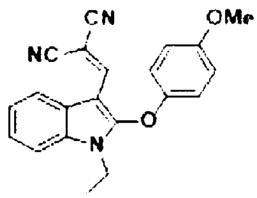 |
| A6 | 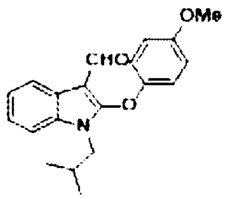 | Compound 6 | 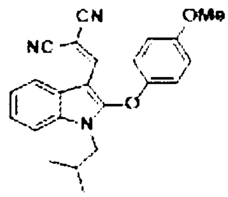 |
| A7 | 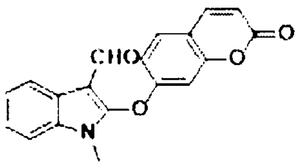 | Compound 7 | 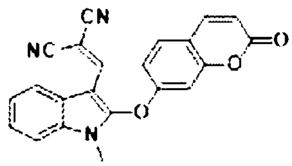 |
| A8 | 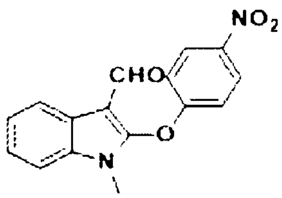 | Compound 8 | 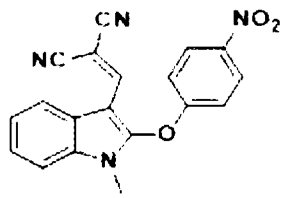 |
| A9 | 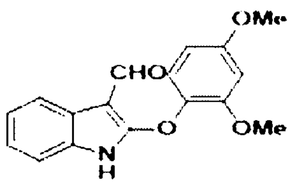 | Compound 9 | 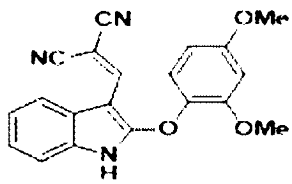 |

(continued)

| P-1 Compound | | P Compound | |
|---|---|---|---|
| A 10 | 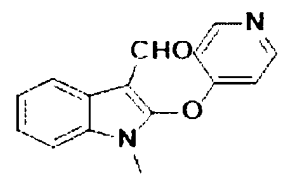 | Compound 10 | 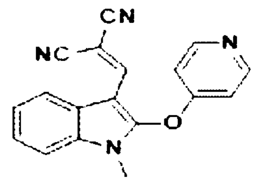 |
| A11 | 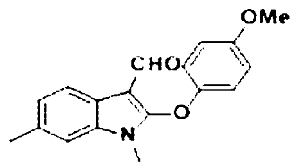 | Compound 11 | 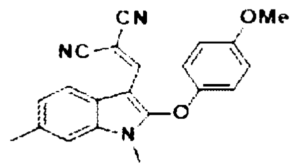 |
| A12 | 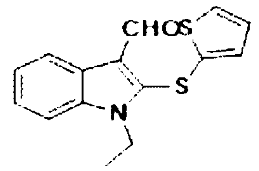 | Compound 12 | 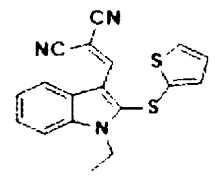 |
| A 13 | 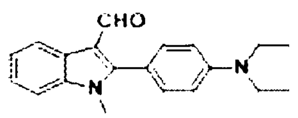 | Comparative Compound 1 | 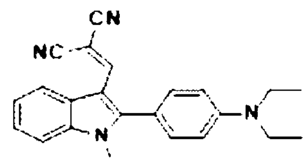 |

**<Examples 1 to 8 and Comparative Example 1> Preparation of adhesive composition**

Based on 100 parts by weight a solid content except for a solvent in an adhesive composition, 95.4 parts by weight of an adhesive material (AD-701 solid content, LG Chem.), 0.29 parts by weight of Compound 1, 0.10 parts by weight of an isocyanate-based cross-linking agent (T39M, Soken Chemical & Engineering Co., Ltd.), 0.2 parts by weight of a silane coupling agent (T-789J, Soken Chemical & Engineering Co., Ltd.), 0.5 parts by weight of an antioxidant (Kinox-80, Hannong Chemicals Inc.), 1.5 parts by weight of an antistatic agent (FC-4400, 3M), 2 parts by weight of a light stabilizer (Tinuvin 123, BASF SE), and 0.01 parts by weight of a catalyst (dibutyltin dilaurate, Sigma-Aldrich) in the following Table 2 were added to the adhesive composition, the solvent (EA) was added thereto in an amount of 25 parts by weight based on the total parts by weight of the adhesive composition, and the resulting mixture was mixed using a mixer (Shaker, SKC 6100, JEIO Tech.), thereby preparing an adhesive composition.

The following Table 2 shows the content based on 100 parts by weight of the solid content except for the solvent in the adhesive composition.

[Table 2]

| | Adhesive material (parts by weight) | Compound (parts by weight) | | Cross-linking agent (parts by weight) | Silane coupling agent (parts by weight) | Anti oxidant (parts by weight) | Antistatic agent (parts by weight) | Light stabilizer (parts by weight) | Catalyst (parts by weight) |
|---|---|---|---|---|---|---|---|---|---|
| Comparative Example 1 | 95.4 | Comparative Compound 1 | 0.29 | 0.10 | 0.20 | 0.50 | 1.50 | 2.0 | 0.01 |
| Example 1 | 95.4 | Compound 1 | 0.29 | 0.10 | 0.20 | 0.50 | 1.50 | 2.0 | 0.01 |
| Example 2 | 95.4 | Compound 2 | 0.29 | 0.10 | 0.20 | 0.50 | 1.50 | 2.0 | 0.01 |
| Example 3 | 95.4 | Compound 3 | 0.29 | 0.10 | 0.20 | 0.50 | 1.50 | 2.0 | 0.01 |
| Example 4 | 95.4 | Compound 4 | 0.29 | 0.10 | 0.20 | 0.50 | 1.50 | 2.0 | 0.01 |

(continued)

| | Adhesive material (parts by weight) | Compound (parts by weight) | | Cross-linking agent (parts by weight) | Silane coupling agent (parts by weight) | Anti oxidant (parts by weight) | Antistatic agent (parts by weight) | Light stabilizer (parts by weight) | Catalyst (parts by weight) |
|---|---|---|---|---|---|---|---|---|---|
| Example 5 | 95.4 | Compound 5 | 0.29 | 0.10 | 0.20 | 0.50 | 1.50 | 2.0 | 0.01 |
| Example 6 | 95.4 | Compound 6 | 0.29 | 0.10 | 0.20 | 0.50 | 1.50 | 2.0 | 0.01 |
| Example 7 | 95.4 | Compound 7 | 0.29 | 0.10 | 0.20 | 0.50 | 1.50 | 2.0 | 0.01 |
| Example 8 | 95.4 | Compound 8 | 0.29 | 0.10 | 0.20 | 0.50 | 1.50 | 2.0 | 0.01 |

**<Experimental Examples>**

Experimental Example 1: Measurement of maximum absorption wavelength of adhesive composition and adhesive film

The adhesive compositions of Examples 1 to 8 and Comparative Example 1 were each applied onto a PET base, and then a film was formed. Next, the adhesive film was detached from a glass substrate, and the maximum absorption wavelength and light resistance of the thin film were confirmed. The following Table 3 shows the results of the maximum absorption wavelengths (Amax (solution)) for the adhesive compositions of Examples 1 to 8, the maximum absorption wavelengths (Amax (thin film)) for adhesive films prepared by the adhesive compositions and the light resistances thereof.

**Experimental Example 2: Evaluation of light resistance of adhesive film**

A release layer (PET) was coated with each of the adhesive compositions of Examples 1 to 8 and Comparative Example 1 to a thickness of 22 μm to 23 μm using a knife bar coating apparatus (KP-3000, Kipae E&T Co., Ltd.) to prepare an adhesive film. After coating, the release layer was removed, and the adhesive film and a binder resin film (TAC: cellulose triacetate) were sequentially stacked on glass through lamination, thereby producing a sample.

Immediately after producing the sample, the light resistance was evaluated by measuring the transmittance of the sample to light with a wavelength of 390 to 450 nm using an UV-vis apparatus (Shimazu UV-3600). After each of the samples was exposed to a light intensity of 55,600 lx for 48 hours, the transmittance to light with a wavelength of 390 to 450 nm was re-measured. ΔT was calculated by dividing the value obtained by subtracting the transmittance immediately after the sample was produced from the re-measured transmittance by the transmittance value immediately after the sample was produced, and the results under the evaluation conditions are shown in the following Table 3.

[Table 3]

| | Compound | Λmax (solution) | Λmax (thin film) | Light resistance △T % |
|---|---|---|---|---|
| Example 1 | Compound 1 | 384 | 386 | 6.46 |
| Example 2 | Compound 2 | 396 | 395 | 6.04 |
| Example 3 | Compound 3 | 385 | 386 | 0.89 |
| Example 4 | Compound 4 | 387 | 388 | 0.92 |
| Example 5 | Compound 5 | 388 | 387 | 0.87 |
| Example 6 | Compound 6 | 388 | 387 | 0.85 |
| Example 7 | Compound 7 | 385 | 386 | 2.55 |
| Example 8 | Compound 8 | 385 | 386 | 3.30 |
| Comparative Example 1 | Comparative Compound 1 | 403 | 404 | 17.7 |

However, in Table 3 above, the evaluation solvent was EA, and the light resistance was evaluated by irradiating with an LED lamp under the light intensity and time conditions.

From the experimental results in Table 3 above, it can be confirmed that according to Examples 1 to 8, while

satisfying a target wavelength range in the solution (adhesive composition) state and the thin film (adhesive film) state, light resistance was improved by introducing a linking group (linker) such as O or S between indoline and phenyl, and that the presence of a substituent capable of transferring electrons (electron donating group) rather than a substituent that withdraws electrons (electron-withdrawing group) in phenyl has a greater effect on improving light resistance. In contrast, it can be confirmed that according to Comparative Example 1, even though the target wavelength range is satisfied in the solution (adhesive composition) state and the thin film (adhesive film) state, the light resistance is poor at 17.7% due to a structure in which indoline and phenyl are directly bonded.

## Claims

1. A compound represented by the following Chemical Formula 1:

[Chemical Formula 1]

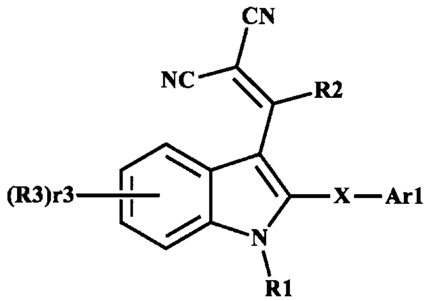

wherein, in Chemical Formula 1,

R1 to R3 are the same as or different from each other, and are each independently hydrogen; a halogen group; or a substituted or unsubstituted alkyl group,
X is O or S,
Ar1 is a substituted or unsubstituted aryl group; or a substituted or unsubstituted heteroaryl group, and
r3 is an integer from 0 to 4, and when r3 is 2 or higher, R3's are the same as or different from each other.

2. The compound of claim 1, wherein R1 is hydrogen; or a substituted or unsubstituted alkyl group having 1 to 10 carbon atoms.

3. The compound of claim 1, wherein R2 is hydrogen.

4. The compound of claim 1, wherein R3 is hydrogen; a halogen group; or a substituted or unsubstituted alkyl group having 1 to 10 carbon atoms.

5. The compound of claim 1, wherein Ar1 is a substituted or unsubstituted aryl group having 6 to 30 carbon atoms; or a substituted or unsubstituted heteroaryl group having 2 to 30 carbon atoms.

6. The compound of claim 1, wherein Chemical Formula 1 is represented by any one of the following compounds:

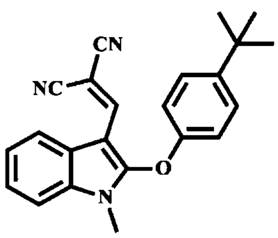
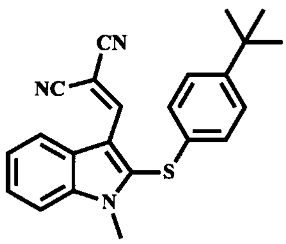
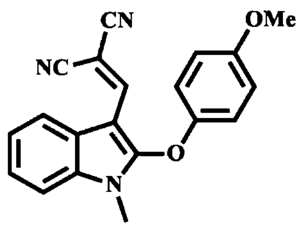

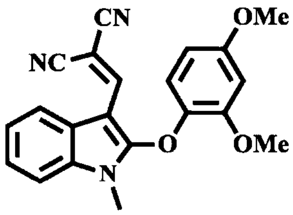
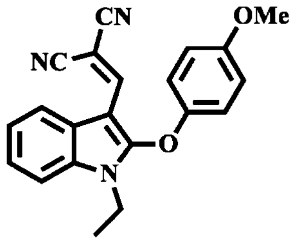
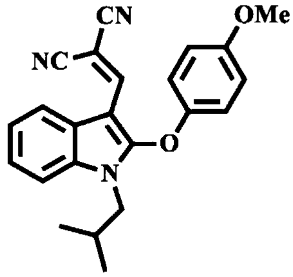

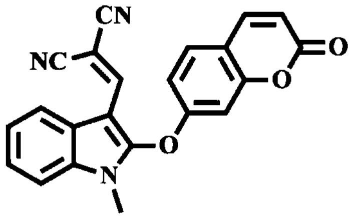
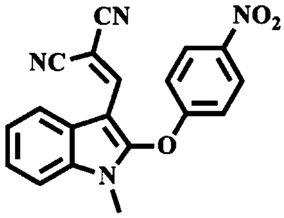

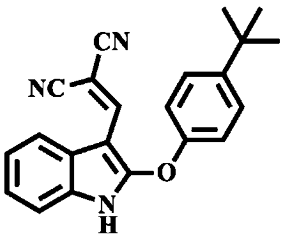
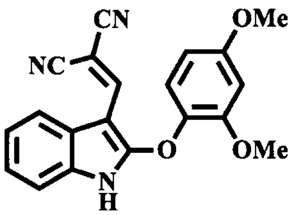
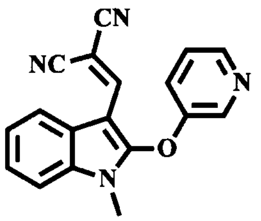

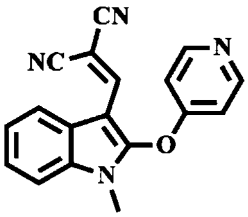
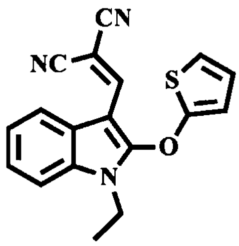
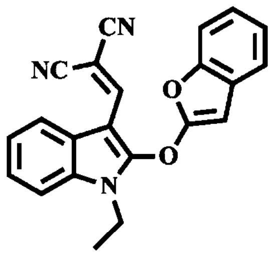

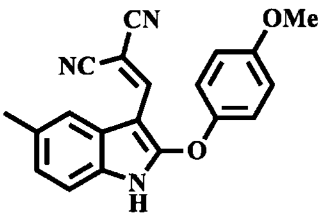
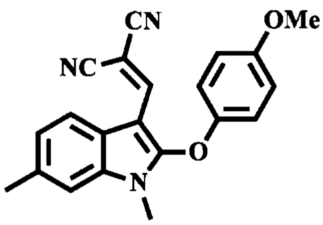
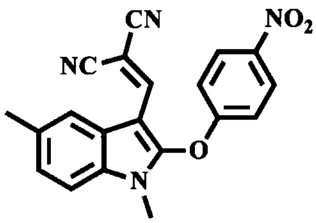

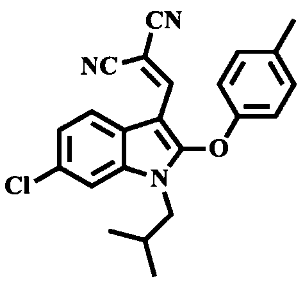
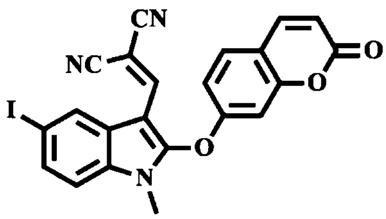
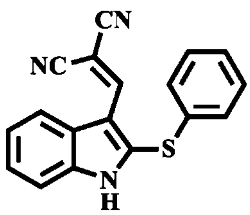

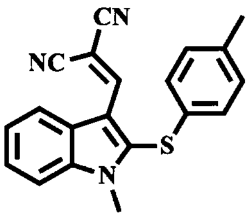
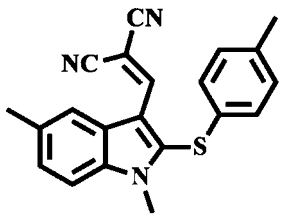
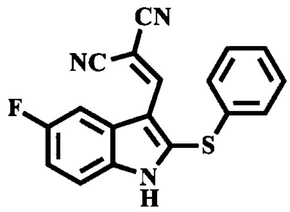

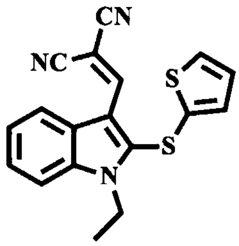
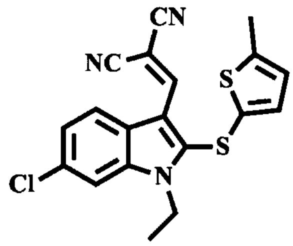

**7.** An adhesive composition comprising the compound of any one of claims 1 to 6; and an adhesive material.

**8.** The adhesive composition of claim 7, wherein a content of the adhesive material is 93 to 97 parts by weight based on 100 parts by weight of a solid content of the adhesive composition.

**9.** The adhesive composition of claim 7, wherein a content of the compound is 0.10 to 0.50 parts by weight based on 100 parts by weight of a solid content of the adhesive composition.

**10.** The adhesive composition of claim 7, further comprising one or more selected from a cross-linking agent, an antioxidant, an antistatic agent, a silane coupling agent, a light stabilizer, and a catalyst.

**11.** The adhesive composition of claim 7, wherein the adhesive composition exhibits the maximum absorption wavelength in a wavelength range of 380 nm or more and 420 nm or less.

**12.** An adhesive film comprising the adhesive composition according to claim 7.

**13.** The adhesive film of claim 12, wherein the adhesive film exhibits the maximum absorption wavelength in a wavelength range of 380 nm or more and 420 nm or less.

**14.** The adhesive film of claim 12, wherein the adhesive film satisfies the following Equation (1):

$$10\ \% \geq |T_0 - T_i| \quad \text{- Equation (1)}$$

in Equation (1),

$T_0$ is a transmittance value in unitsof % measured before irradiation by coating the adhesive film with a thickness of 20±3 μm, and
$T_i$ is a transmittance value in units of % measured after irradiating the adhesive film with a light intensity of 55,600 lx for 48 hours.

**15.** An electronic device comprising the adhesive film according to claim 12.

[Figure 1]

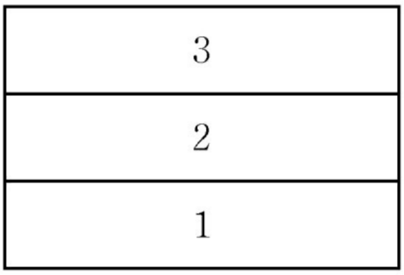

**INTERNATIONAL SEARCH REPORT**

International application No.
**PCT/KR2023/014221**

**A. CLASSIFICATION OF SUBJECT MATTER**

**C07D 209/30**(2006.01)i; **C07D 209/34**(2006.01)i; **C07D 405/12**(2006.01)i; **C07D 401/12**(2006.01)i; **C07D 409/12**(2006.01)i; **C09J 11/06**(2006.01)i; **C09J 7/30**(2018.01)i; **C08K 5/3417**(2006.01)i; **C08K 5/45**(2006.01)i; **C08K 5/372**(2006.01)i

According to International Patent Classification (IPC) or to both national classification and IPC

**B. FIELDS SEARCHED**

Minimum documentation searched (classification system followed by classification symbols)

C07D 209/30(2006.01); C09J 133/08(2006.01); G02B 1/14(2015.01); G02B 5/28(2006.01); G02B 5/30(2006.01); G02F 1/13363(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 인돌(indole), 사아노비닐기(cyanovinyl group), 점착필름(adhesive film), 전자소자(electronic device)

**C. DOCUMENTS CONSIDERED TO BE RELEVANT**

| Category* | Citation of document, with indication, where appropriate, of the relevant passages | Relevant to claim No. |
|---|---|---|
| X<br>A | Chemical abstract compounds. STNext.<br>RN 771568-95-7 (Entered STN: 29 October 2004), RN 771568-66-2 (Entered STN: 29 October 2004). | 1-5<br>6-15 |
| A | KR 10-2017-0095147 A (SUMITOMO CHEMICAL CO., LTD.) 22 August 2017 (2017-08-22)<br>See claims 1-16; and paragraphs [0161]-[0171]. | 1-15 |
| A | KR 10-2017-0077817 A (SUMITOMO CHEMICAL CO., LTD.) 06 July 2017 (2017-07-06)<br>See claims 1-10; and paragraphs [0125]-[0135]. | 1-15 |
| A | KR 10-2022-0119464 A (SUMITOMO CHEMICAL CO., LTD.) 29 August 2022 (2022-08-29)<br>See claims 1-24. | 1-15 |
| A | WO 2017-159969 A1 (SAMSUNG SDI CO., LTD.) 21 September 2017 (2017-09-21)<br>See claims 1-29. | 1-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 January 2024** | **15 January 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2023/014221**

| Patent document cited in search report | Publication date (day/month/year) | Patent family member(s) | Publication date (day/month/year) |
|---|---|---|---|
| KR 10-2017-0095147 A | 22 August 2017 | CN 107085257 A | 22 August 2017 |
| | | CN 107085257 B | 01 June 2021 |
| | | JP 2017-142412 A | 17 August 2017 |
| | | JP 6777401 B2 | 28 October 2020 |
| | | TW 201741423 A | 01 December 2017 |
| | | TW I744282 B | 01 November 2021 |
| | | US 2017-0235032 A1 | 17 August 2017 |
| KR 10-2017-0077817 A | 06 July 2017 | CN 106990472 A | 28 July 2017 |
| | | CN 106990472 B | 19 February 2021 |
| | | JP 2017-120430 A | 06 July 2017 |
| | | JP 6829070 B2 | 10 February 2021 |
| | | TW 201738349 A | 01 November 2017 |
| | | TW I731012 B | 21 June 2021 |
| | | US 10690824 B2 | 23 June 2020 |
| | | US 2017-0184766 A1 | 29 June 2017 |
| KR 10-2022-0119464 A | 29 August 2022 | CN 114867811 A | 05 August 2022 |
| | | JP 2021-105167 A | 26 July 2021 |
| | | TW 202132495 A | 01 September 2021 |
| | | WO 2021-132235 A1 | 01 July 2021 |
| WO 2017-159969 A1 | 21 September 2017 | CN 108885372 A | 23 November 2018 |
| | | CN 108885372 B | 08 October 2021 |
| | | KR 10-1813813 B1 | 29 December 2017 |
| | | KR 10-2017-0108792 A | 27 September 2017 |
| | | KR 10-2017-0122162 A | 03 November 2017 |
| | | KR 10-2017-0122163 A | 03 November 2017 |
| | | TW 201739812 A | 16 November 2017 |
| | | TW I649364 B | 01 February 2019 |
| | | US 10816710 B2 | 27 October 2020 |
| | | US 2019-0107657 A1 | 11 April 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020220184448
- JP 2012211305 A